(19) Europäisches Patentamt
European Patent Office
Office européen des brevets

(11) Publication number: 0 006 313 B1

# (12) EUROPEAN PATENT SPECIFICATION

(45) Date of publication of patent specification: 22.09.82

(51) Int. Cl.[3]: C 07 C 31/18, C 07 C 31/26, C 07 C 29/136 //B01J23/72

(21) Application number: 79300949.9

(22) Date of filing: 25.05.79

## (54) Process for the reduction of sugars to sugar alcohols.

(30) Priority: 25.05.78 GB 2243178

(43) Date of publication of application: 09.01.80 Bulletin 80/1

(45) Publication of the grant of the patent: 22.09.82 Bulletin 82/38

(84) Designated Contracting States: AT BE CH DE FR GB IT LU NL SE

(56) References cited:
DE - A - 1 667 106
DE - B - 2 538 253
DE - C - 544 666
FR - A - 2 256 780
GB - A - 1 022 480
GB - A - 1 025 813
US - A - 2 224 011
US - A - 2 818 851

(73) Proprietor: IMPERIAL CHEMICAL INDUSTRIES PLC
Imperial Chemical House Millbank
London SW1P 3JF (GB)

(72) Inventor: Dewing, John
59 Dee Banks
Chester Cheshire, CH3 5UU (GB)
Inventor: Ruddlesden, Jane Frances
28 Silverdale Close
Frodsham Cheshire (GB)
Inventor: Stewart, Allan
20 Arran Drive
Frodsham Cheshire (GB)
Inventor: Thompson, David John
50 Marle Croft Old Hall Park
Whitefield Lancashire (GB)

(74) Representative: Bate, Bernard James et al,
Imperial Chemical Industries PLC Legal Department: Patents Thames House North Millbank
London SW1P 4QG (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for the reduction of sugars to sugar alcohols

This invention relates to a process for the reduction of sugars to sugar-alcohols.

It is known that a variety of reducing agents and catalysts may be used to hydrogenate sugars to sugar alcohols but the majority are not stereo-selective. The sugar alcohols which are produced are a polyol with the same number of carbon atoms as the original sugar and all the carbon atoms having a hydroxyl group attached. During the process of reduction of a keto-sugar to a sugar alcohol the carbonyl group of the sugar is hydrogenated to a secondary alcohol group, the carbon atom of which is a chiral centre and the secondary alcohol group has R- and S-forms. In the reduction of a particular keto-sugar most reducing agents and catalysts produce equal proportions of the R and S forms by hydrogenation and thus equal amounts of the two different stereo-isomeric products result.

GB 354196 describes the use of nickel, copper or cobalt catalysts for the hydrogenation of aldoses (e.g. glucose to sorbitol) but makes no mention of the catalytic hydrogenation of ketoses.

DE—OS 544,666 describes the catalytic hydrogenation of fructose to the corresponding polyhydric alcohols in the presence of a supported nickel catalyst.

GB 1022480 refers to catalytic hydrogenation prior art processes in which glucose is converted to sorbitol and fructose is converted to mannitol and sorbitol (in equal proportions), and describes a process for increasing the proportion of mannitol obtained. This involves a two-step process for converting an aqueous mixture of glucose and fructose (e.g. invert sugar) to mannitol and sorbitol in which (1) the fructose portion is hydrogenated in the presence of a nickel catalyst under neutral conditions to give mannitol and sorbitol without substantial hydrogenation of the glucose portion, and (2) the glucose portion is isomerised to fructose under alkaline conditions, e.g. by the addition of lime, and the fructose thus obtained is then catalytically hydrogenated to give mannitol and sorbitol. Up to 36.9% mannitol may be obtained in the mannitol/sorbitol product mixture as a result of the two-stage hydrogenation and isomerisation processes.

GB 1025813 describes a combined process involving both isomerisation and hydrogenation. Glucose and/or invert sugar is hydrogenated in the presence of lime and a supported nickel catalyst, which optionally may contain copper, to give a product containing mannitol and sorbitol. Glucose (or the glucose fraction of invert sugar) is preferentially isomerised to fructose rather than being hydrogenated to sorbitol, and the fructose thus obtained is hydrogenated to give mannitol and sorbitol. The overall effect of the combined process is to increase the proportion of mannitol.

DE—OS 1931112 describes a combined process involving hydrolysis of saccharose to fructose and glucose, and catalytic hydrogenation of the hydrolysis products to mannitol and sorbitol. Conventional hydrogenation catalysts are used e.g. supported nickel, which may contain iron and copper as suitable activators, under conditions of pH equal to 6—8 and a reaction temperature above 160°C, viz. 160°C—190°C, e.g. 160°C—175°C. High mannitol yields (up to 31%) are obtained, which under the conditions used, could involve isomerisation of glucose to fructose.

We have now found an improved process by which the reduction may be conducted so as to produce a considerable excess of one stereo isomeric product and ideally one stereo isomeric product exclusively. This improved process also gives very high yields of the sugar alcohols relative to the sugar converted to other products when carried out at temperatures known to cause degradation and other side reactions in other hydrogenation processes.

According to the present invention a process for the production of a sugar alcohol by the hydrogenation of a keto-sugar comprises contacting, in a pressurised system containing hydrogen gas at a temperature of at least 80°C, a solution of the keto-sugar with a supported catalyst comprising finely divided metal and a particulate support material, characterised in that the keto-sugar is stereo-selectively hydrogenated by using a catalyst comprising finely divided metallic copper and a particulate support material, and wherein the individual particles of copper have a maximum dimension of less than 60 nm, more suitably less than 35 nm. The preferred example is a catalyst consisting of individual particles of copper metal dispersed over the surface of the support material including a catalyst wherein metallic copper is dispersed throughout a porous vehicle.

The support material or porous vehicle is preferably an insoluble substrate i.e. a solid which is insoluble or only sparingly soluble in the solvent which is used in the solution of the keto-sugar and which is stable under the reducing conditions used to make the catalyst. The preferred substrates are particulate solids conventionally used as catalyst support materials for example alumina, silica, kieselguhr, silica-alumina, graphite or molecular sieves and of these we especially prefer silica or kieselguhr. The surface area of a solid substrate for the copper may be for example from 5 sq m/g to 1000 sq m/g, preferably ca. 200—500 sq m/g if silica is used.

The term keto-sugar is used to denote a saccharide molecule containing a single keto group including for example keto-tetroses, -pentoses and -hexoses, e.g. xylulose, fructose or sorbose and does not exclude mixtures of ketoses with aldoses, an example of such mixtures being invert sugar.

The particles of the catalyst comprising copper metal may be produced by conventional methods of deposition of metal for example by vacuum evaporation or electrolytically or by methods involving chemical reaction for example in the decomposition of copper compounds, e.g. copper oxalate. The

catalyst is preferably produced by the reduction in the presence of a particulate support material of a dispersed form of a compound of copper for example an oxide, a copper salt of an inorganic or organic acid or a co-ordination compound having inorganic or organic ligands.

The catalyst preparation is suitably conducted by chemical reduction of a dispersed form of the compound of copper in the presence of the particulate support material at an elevated temperature e.g. from 100°C to 500°C in a non-oxidising atmosphere for example an atmosphere of a rare gas, nitrogen or preferably a reducing atmosphere especially an atmosphere containing hydrogen gas. The particulate metallic copper which results may be deposited on the exterior surface of the support granules or within the pore structure of this support. The metal may still be mixed with unreduced or not fully reduced compounds of copper e.g. an external layer of copper oxide on the metal. It is desirable not to expose the reduced catalyst to air prior to use: however, exposure to air is not as detrimental to use of the catalysts according to this invention as it is in the case of nickel catalysts.

The copper metal may be mixed with minor amounts of another metal or metals especially those of Group VIII of the Periodic Table, e.g. Ni, Pt, Pd. This mixture may be an alloy or a physical mixture of the metals. Such a mixed metal catalyst may be prepared by reduction of mixtures of compounds of copper with compounds of the other metals or by simultaneous deposition of the metals on or with the support.

It is pointed out in the prior art referred to in British Patent No. 1022480 not only that fructose on hydrogenation yields essentially equal proportions of mannitol and sorbitol but also that departure from essentially neutral conditions favours unwanted side reactions. Surprisingly therefore we find both high relative yields of mannitol from fructose hydrogenation and low yields of by-products from the side reactions using the process of this present invention.

The hydrogenation of the keto-sugar is preferably carried out in neutral or acid conditions, i.e. pH not greater than 7. Normally the reactants are initially slightly acidic and during the reaction the pH of the system falls to a value of 4—5. Under such conditions it is surprising to find that the reaction is stereo-selective for the whole range of keto-pentoses and hexoses which we tried and that the products consist of a considerable excess of one stereoisomer.

It has been observed that the selectivity and/or the yield of the process of the invention is often improved if an oxygen-free inorganic anion is present in the system during the hydrogenation. The anions preferred are halogen anions, chloride, bromide and iodide and especially active is the chloride ion. The anions may be added to the system (for example, dissolved in an aqueous medium) in the form of an inorganic or organic salt, for example the salts of alkaki and alkaline earth metals or the higher transition metals (e.g. cobalt or zinc) or even aqueous solutions of the hydrogen halide have been found to be suitable. Similar improvements are observed if boron compounds are present preferably oxyacids or oxyanions of boron especially orthoboric acid or di-sodium tetra-borate: they are conveniently added to the solvent medium in which the process is conducted.

The mode of action of these additives which improve the process is not known in detail: it is not thought to be necessary for the copper metal in the catalyst to react stoichiometrically with the anions because the anionic improvers are effective in very small quantities so that a considerable excess of copper metal will always be present. The proportion of additive used may range from trace amounts up to 50% by weight of the copper in the catalyst, preferably from 0.1% to 25%.

It will be appreciated that for a particulate catalyst the greater the surface area of copper the greater the reactivity and accordingly the catalyst is preferably prepared under conditions which will produce smaller particles of copper. For catalysts prepared by the method described herein under the heading "Catalyst Preparation and Characterisation" it has been found that if reduction of the copper compounds is carried out at a temperature between 300°C and 400°C particles of copper catalyst having an average size within the range 25—35 nm are produced, whereas at a temperature of 450°C a preponderance of larger particles for example up to ca 60 nm is observed.

Since catalyst particle size is dependent upon both reduction temperature and reduction time use of shorter reduction times than those indicated herein should result in smaller particle sizes at any given temperature.

For the process of the present invention it is preferable to use a highly active catalyst especially for reduction on a commercial scale and thus catalysts have been prepared wherein the majority of the copper particles are below 10 nm. For example catalysts prepared from copper salts precipitated with water glass solutions were observed to contain after reduction a majority of individual particles of copper metal having dimensions less than 10 nm and such catalysts exhibited high activity and good selectivity in the reduction of keto-sugars (e.g. Examples 13, 14, 17 and 18).

The keto-sugar is brought into contact with the support copper catalyst in the presence of a solvent medium. The solvent medium may be an organic solvent for sugars but it is preferably a hydrophilic solvent and especially an aqueous solvent medium of which pure water is the most preferred. As described hereinbefore additives giving rise to anions may be conveniently dissolved in the aqueous solvent medium but if desired the additives may be used to pre-treat the catalyst before it is brought into contact with the sugar in the solvent medium.

The ketose used as starting material may be a mixture of stereoisomeric forms but preferably one isomer only is used because the reduction of the keto group in the sugar in the present process occurs

in a stereo-selective manner. A secondary alcohol group is formed from the keto group and it is observed in the process of this present invention that a preponderance of either an R- or an S-chiral centre may be produced at the reduced keto group. Thus the process may be used to produce an excess of one particular stereoisomeric form of the sugar alcohol product for example D-mannitol from fructose or D-glucitol otherwise known as sorbitol from L-sorbose and so on for other sugars as shown in the Examples.

Thus considering straight chain Fischer projections of the relevant carbon atoms involved in the stereochemistry of the reduction of ketoses we can represent the reaction as follows:

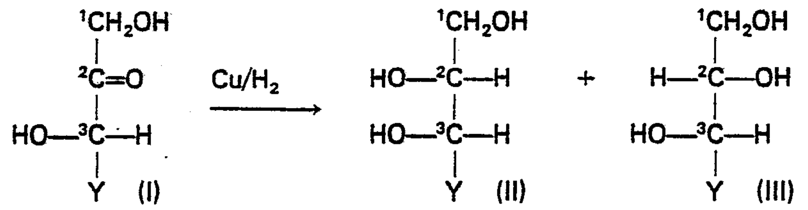

(I) (II) (III)

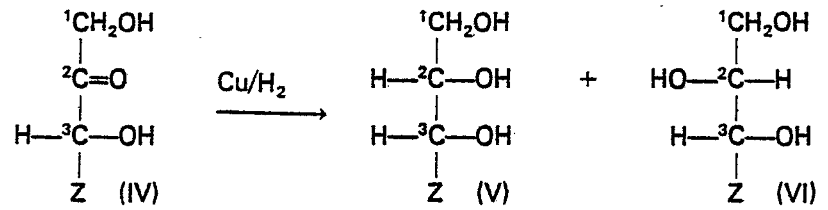

(IV) (V) (VI)

Compounds I and IV are two ketoses possessing different stereo chemical configuration of the substituents on carbon atom 3. If the groups Y and Z contain at least two carbon atoms (i.e. the ketoses are pentoses, hexoses or higher homologues) the stereo isomers of the sugar alcohols found in excess are those represented by II and V while III and VI are observed to be the minor products.

Thus a keto-pentose or keto-hexose with a R-configuration at carbon atom-3 (compound I) will produce in the process of this invention a considerable excess of the pentitol or hexitol product respectively which contains an R-configuration at carbon atom-2.

Similarly also those with an S-configuration at carbon atom-3 (compound IV) will give a considerable excess of sugar alcohol products which have an S-configuration at carbon atom-2. (The assignment of the R- and S-configuration is as described in "Organic Chemistry" by D. J. Cram & G. S. Hammond 2nd Edition published by McGraw-Hill 1964 pp. 179—180).

This general pattern for the stereo selectivity of the copper catalyst system used in this invention seems to apply to all ketoses although for those containing only four carbon atoms (tetroses) the stereo specific nomenclature will be different.

The conditions of the operation of the process may be adjusted in order to enhance the yield of the desired product. For example we prefer to operate the hydrogenation process at a temperature between 80°C and 200°C especially between 80°C and 170°C.

The pressure of the system is always in excess of atmospheric, normally the reaction is carried out using a pressure of gas containing hydrogen of 10 to 1,000 bars, conveniently in the range from 30 to 300 bars.

Catalyst preparation and characterisation

One preferred method of preparing the catalyst is described hereinafter whereby batches were prepared by the following procedure.

34.2 g of cupric nitrate trihydrate was dissolved in 300 ml of deionised water. 22 g of Davison 952 silica, used as supplied having a surface area of 300—350 $m^2/g$ was added and mixed thoroughly. The temperature of the solution was raised to 40—50°C and a solution of 57.4 g ammonium bicarbonate in 500 ml deionised water was added dropwise with stirring over a period of one hour. The mixture was then stirred and boiled for a further hour. Then the greenish-blue solid formed was filtered off, washed with 4 litres of deionised water and then dried at 160°C in a vacuum oven for 3 hours. Prior to use as a catalyst, 1—2 g batches of the solid prepared as above were each reduced by heating up to a chosen temperature between 300°C and 550°C over a period of between 1/4 and 1/2 hour in a vitreous silica furnace tube containing a flow of reducing gas consisting of 10% hydrogen in nitrogen at a flow rate of 50 ml/min. The chosen temperature was maintained for 6 hours while the same flow of the reducing gas mixture was continued. After this time, the heating was stopped and once the apparatus and the solid therein cooled to room temperature, the solid was removed for use or examination. Except for manipulation during characterisation or use the catalyst was maintained at all times in a nonoxidising atmosphere e.g. of nitrogen or hydrogen/nitrogen mixtures.

Samples of solid, prepared and reduced as described above at the chosen reduction temperature

of 350°C were examined by electron microscopy and electron diffraction. Electron microscopy revealed that the solid consisted of particles of the support material, the surface of which was largely coated with smaller particles, close to spherical in shape, many of which had diameters of about 25 nm. The preponderance of crystalline material in the solid was copper metal, as identified by electron diffraction. The first thirteen allowed reflexions for an f.c.c. material were observed as rings from areas about 10 microns in diameter. By reference to a polycrystalline silver standard, the lattice parameter was measured to be $0.361 \pm 0.003$ nm, which compares well with two values 0.36147 and 0.36504 nm given for copper metal in "Crystal Data Determinative Tables" 2nd Edition 1963, published by the American Crystallographic Association.

Another analysis experiment was carried out on a batch reduced in the same way to that described above. The batch was further reduced at a higher temperature of 500°C and the reduction was conducted in a microbalance in a flowing mixture of 10% hydrogen in nitrogen after previously being taken to constant weight at 350°C in $N_2$ alone. A weight loss of only 0.3% was observed during the second reduction showing that the majority of the sample was fully reduced in the first reduction. This indicates that large concentrations of copper oxides or salts are absent in samples reduced in the manner described for the catalyst preparation. If it is assumed that the small weight loss of 0.3% was due to the removal of a monolayer of bound oxygen on the metallic phase, the surface area of metal would be consistent with the electron microscopy observations and the copper analysis of 21.8% in the solid.

This copper catalyst has been found to be more stable in use and therefore easier to handle than conventional nickel catalysts used hitherto for the selective hydrogenation of sugars. The stability to the atmosphere of the catalysts used in the present invention has allowed us to store them for up to a few days before use, preferably under an atmosphere of hydrogen. Example 18 demonstrates that the stability of the copper catalyst allows re-cycle of catalyst in batch operation with only slight loss in activity which is remarkable since the catalyst has simply been filtered from the discharged reduction product mixture in the open laboratory and returned to the autoclave with a fresh charge of sugar syrup. Prolonged active life of a selective copper catalyst is a great advantage in a manufacturing process because the down-time for regeneration of catalyst contributes significantly to the cost of the process. Therefore the process of this invention is easier and cheaper to operate than prior art processes for at least two reasons, the catalyst stability and the greater selectivity of the process especially with additives.

The invention is illustrated by the following examples. All analyses are given in percentage by weight on the dry basis.

Example 1

1 g of reduced catalyst prepared as hereinbefore particularly described in the section headed "Catalyst Preparation and Characterisation" and at a chosen temperature of 450°C was added to a Pyrex (a Registered Trade Mark) autoclave liner containing a solution of 6.25 g D-fructose in 25 ml of deionised water and also a magnetic stirring bar coated with PTFE. This mixture of catalyst and sugar in water was placed in its liner into a 100 ml stainless steel autoclave, which was then flushed with hydrogen gas and sealed before pressurizing with hydrogen to 103 bars. The autoclave and its contents were stirred, heated for about 10 minutes until a reaction temperature of 100°C was reached and this temperature was maintained at 100°C for a further 2 hours whilst stirring was continued. Heating was then stopped and the autoclave and its contents allowed to cool before the gases were vented. The liner and its contents were removed from the opened autoclave and the liner sealed with a rubber seal. Samples of the supernatant liquid were removed and derivatives of the carbohydrate products and/or unreacted fructose were prepared for g.l.c. analysis so that the extent of conversion of the original sugar and selectivity of the reaction could be ascertained.

The analytical method used was as follows wherein two derivatives of the reduced sugars namely (a) the trimethylsilyl and (b) the acetate derivative were prepared and analysed by gas/liquid chromatography.

(a) Preparation and measurement of trimethylsilyl derivatives (TMS method)

A sample of the supernatant liquid (5—15 mg dry weight equivalent) was pipetted into a screw-cap septum vial. A solution containing hydroxylamine hydrochloride (25 mg) in AR pyridine (1 ml) was then added through the septum and the solution heated at 70°C for 30 minutes. After cooling to room temperature trimethylsilylimidazole (1 ml) was added, the vial shaken and then allowed to stand at room temperature for a further 30 minutes. 1 $\mu l$ of this solution was then analysed by g.l.c. on a 1.5 m×3 mm glass column packed with 3% silicone OV-17 on "Chromosorb" W(HP) (80—100 mesh) maintained at 130°C for 10 minutes after injection before a programmed temperature rise of 2°C/min to a final temperature of 210°C.

(b) Preparation and measurement of acetate derivatives (AC method)

The sugar mixture (5—15 mg dry weight) in a screw-cap septum vial was treated with a solution of hydroxylamine hydrochloride (25 mg) in AR pyridine (1 ml) and then acetic anhydride (1 ml). This

mixture was then heated at 90°C for 1 hour. 1 µl of this solution was then analysed by g.l.c. on a 1.5 m×3 mm glass column packed with 5% silicone gum rubber XE-60 on "Chromosorb" W(HP) (80—100 mesh) maintained at 190°C for 16 minutes, after injection, before a programmed temperature rise of 16°C/min to a final temperature of 210°C.

From the foregoing analysis by the TMS method the % conversion was measured as 98. The hexitol products were analysed as

| | % D-mannitol | % D-glucitol (Sorbitol) |
|---|---|---|
| TMS method | 65 | 33 |
| AC method | 68 | 32 |

Note: Silicone OV17 on "Chromosorb" W supplied by Pierce and Warriner (UK) Limited. Silicone gum rubber XE-60 on "Chromosorb" W is supplied by Phase Sep Limited.

Example 2

A hydrogenation reaction was carried out according to the method of Example 1 except that sorbose was the sugar solution hydrogenated and only 1 g was used. From analysis by the TMS method the % conversion was measured to 100. The hexitol products were analysed as:

| | % D-Glucitol (Sorbitol) | % L-iditol |
|---|---|---|
| TMS method | 62 | 38 |
| AC method | 68 | 32 |

Example 3

The reaction was carried out in a similar way to that described in Example 1 except that D-ribulose was the sugar solution hydrogenated and only 100 mg of it were used.

The pentitols obtained from the hydrogenation were analysed by g.l.c. of their acetate derivatives by a method similar to that described in Example 1 method (b) except that the column packing was 3% silicone OV-17 on "Chromosorb" W(HP) (80—100 mesh) and was maintained at 170°C for 10 minutes after injection, before a programmed temperature rise of 2°/min to a final temperature of 210°C.

| % Conversion | % Ribitol | % D-Arabitol |
|---|---|---|
| 100 | 69 | 31 |

Example 4

The reaction was carried out in a similar way to that described in Example 1 except that the catalyst was added to a solution of 6.25 g D-fructose in 25 ml of deionised water, to which 14.8 mg of boric acid had previously been added.

| | | Hexitol Products | |
|---|---|---|---|
| | % Conversion | % D-Mannitol | % D-Glucitol (sorbitol) |
| TMS method | >97 | 78 | 22 |
| AC method | | 82 | 18 |

Example 5

A hydrogenation was conducted according to the method described in Example 1 except that the catalyst was added to a solution of 1 g L-sorbose instead of 6.25 g fructose as the ketose solution hydrogenated and to this solution 14.8 mg of ortho-boric acid were added.

| | | Hexitol Products | |
|---|---|---|---|
| | % Conversion | % D-Glucitol (Sorbitol) | % L-Iditol |
| TMS method | 100 | 72 | 28 |
| AC method | | 76 | 24 |

Example 6

The hydrogenation reaction was conducted by the method described in Example 1 except that the catalyst was added to a solution of 6.25 g D-fructose in 25 ml of deionised water to which 15.3 mg of potassium chloride had previously been added.

| | | Hexitol Products | |
|---|---|---|---|
| | % Conversion | % D-mannitol | % D-Glucitol (Sorbitol) |
| TMS method | 39 | 73 | 27 |
| AC method | | 75 | 25 |

Example 7

For this example 1 g of catalyst was reduced at the chosen reduction temperature of 350°C. After reduction the catalyst was left in a furnace tube exposed to air for 3.5 days. It was then added to a solution of 6.25 g D-fructose in 25 ml deionised water and the hydrogenation of the sugar solution was carried out in the same way as described in Example 1 except that the autoclave and its contents were maintained at 100°C for 16 hours. The results were as follows:

| | % Conversion | % D-mannitol | % D-Glucitol (Sorbitol) |
|---|---|---|---|
| TMS method | 62 | 65 | 35 |

Example 8

A catalyst was prepared by the method described in the section entitled "Catalyst Preparation and Characterisation" except that Kieselguhr White from BDH Chemicals Ltd. was used as the support material instead of the silica. Prior to use as a catalyst 1 g of the solid was reduced at the chosen reduction temperature of 350°C. The hydrogenation of D-fructose was conducted using the 1 g of reduced material by the method described in Example 1, except that the temperature of 100°C was maintained for 16 hours rather than 2 hours. The results of the hexitol product analysis by the TMS method were:

% conversion=83, % D-mannitol=66, % D-glucitol (Sorbitol)=34.

Example 9

The reaction was carried out in a similar fashion to that described in Example 2 except that the catalyst was added to a solution of 1 g sorbose in 25 ml of deionised water, to which 14.8 mg of boric acid had previously been added.

| | | Hexitol Products | |
|---|---|---|---|
| | % conversion | % D-glucitol | % L-iditol |
| TMS method | 100 | 72 | 28 |
| AC method | | 76 | 24 |

Example 10

The reaction was carried out in a similar way to that described in Example 1 except that the catalyst had been reduced at the chosen temperature of 350°C in a stream of pure hydrogen gas passing at approximately 30 ml $mm^{-1}$ for 3 hours, and the reduced catalyst was then added to a solution of 6.25 g D-fructose in 25 ml of deionised water to which 33.1 mg of disodium tetraborate had previously been added. The reaction was allowed to continue at 100°C for 16 hours rather than 2 hours and the hydrogen pressure in the autoclave was set at 41.8 bars rather than 103 bars.

The hexitol products were analysed as

| | % D-mannitol | % D-glucitol |
|---|---|---|
| TMS method | 77 | 23 |
| AC method | 80 | 20 |

Example 11

In each of the reactions covered by the following Table, 1 g of reduced catalyst was used to hydrogenate each particular ketose in a similar manner to that described in Example 1. However, these 1 g samples of reduced catalyst come from several different catalyst preparations. Some catalysts

(designated (a)) were prepared in an identical manner to the catalyst hereinbefore particularly described in the section headed "Catalyst Preparation and Characterisation" while other catalysts designated (b) were prepared in a similar way except that 80 g cupric nitrate trihydrate 53 g ammonium bicarbonate and 100 g Davison 952 silica were used.

Since we found some of the ketoses difficult to obtain, the amount of ketose hydrogenated was often significantly less than that described in Example 1 for D-fructose but the experiments were satisfactory in demonstrating the good selectivities in all cases. The table following gives the selectivities to the hexitol or pentitol products for hydrogenations at 100°C as measured by g.l.c. analysis of derivatives.

| Ketose Hydrogenated | Catalyst | Hexitol Products — (% of Hexitols) |
|---|---|---|
| D-Fructose | (a) | D-Mannitol (68) D-glucitol (32) |
| L-Sorbose | (a) | D-glucitol (68) L-iditol (32) |
| D-Tagatose | (a) | D-Talitol (74) galactitol (26) |
| D-Psicose | (b) | D-Allitol (62) Altritol (38) |
| | | Pentitol Products — (% of Pentitols) |
| D-Ribulose | (b) | Ribitol (69) D-Arabitol (31) |

Example 12

This example illustrates the use of a copper hydrogenation catalyst supported on a large-pore zeolite in its sodium form. The counter-ions of the zeolite were partly exchanged using a solution containing copper ions and then the copper was reduced by sodium borohydride as detailed in the following description of the catalyst preparation.

3 g of Na—Y zeolite powder was placed in a solution of 3.7 g cupric acetate in 30 ml deionised water and the mixture refluxed for one hour. After the mixture had cooled it was filtered and washed thoroughly with deionised water. The solid remaining was dried and was analysed. The analysis showed 2.6 wt% Cu, 6.8 wt% Al and 17.8 wt% Si.

1 g of the dried material was suspended in 5 ml deionised water and reduced with stirring by the dropwise addition of a solution of 0.1 g sodium borohydride in 5 ml deionised water under an atmosphere of nitrogen. The resulting black solid was washed with deionised water before use. After the above reduction the black solid was placed in an autoclave and the hydrogenation of D-fructose was carried out in an identical manner to Example 1 except that the reduced copper zeolite material was used as catalyst and the time at 100°C was 4 hours rather than 2 hours.

From analysis by the TMS method described in Example 1 the % conversion was measured as 75% approximately and the hexitol products were analysed as 66% D-mannitol 34% D-glucitol (Sorbitol).

Example 13

A batch of catalyst was prepared from Davison 952 silica, a solution of copper nitrate and a waterglass solution containing 5.3% Na and 10.8% Si as follows:

Two solutions were prepared for use in making a catalyst. One was a solution of 100 g cupric nitrate trihydrate dissolved in deionised water and made up to 500 ml with deionised water. The other was a solution of waterglass made from a more concentrated solution, analysed to contain 5.3% Na and 10.8% Si. The waterglass solution used in the preparation was made by diluting 200 g of this concentrated solution to 500 ml with deionised water.

To carry out the precipitation step, 5 g of Davison 952 silica was suspended in 500 ml of deionised water at room temperature in a large beaker. After calibration an electrode for pH measurement was immersed to the appropriate level in the suspension. Two open glass tubes were positioned with their bases under the level of the liquid in the beaker, and two dropping funnels, one containing the prepared cupric nitrate solution and the other containing the waterglass solution, diluted to 500 ml in the above described manner, were supported so that the effluent from each could drop into glass tubes and run into the suspension in the large beaker, where precipitation could occur. The contents of the beaker were stirred vigorously using an air-powered stirrer mechanism with a stirrer of the type using a half-moon shaped Teflon (a Registered Trade Mark) blade.

The two prepared solutions were added dropwise at the same time to set up and maintain a pH of 5.3 to 6.0 in the contents of the large beaker and to cause the pH to be 5.5 when all the prepared waterglass solution had been added. At this point, about 1.5 hours after the start of the addition of the

solutions, the addition of the cupric nitrate solution was stopped, 335 ml having been added into the contents of the large beaker. Throughout the additions deionised water from a wash bottle was used to wash any solids splashed up on the sides of the large beaker back into the mixture. In all, some 300 ml of additional deionised water was used in this way. After allowing the final mixture about 10 minutes of further stirring, the blue mixture in the large beaker was filtered with suction in a large sintered glass funnel (Pyrex grade 2) and washed thoroughly in the filter with about one litre of deionised water, added in stages after the wet solid had been broken up with a spatula to allow more efficient washing.

The filtered and washed solid was then scraped into a large glass crystallizing dish, which was placed in a vacuum oven. The solid was then dried at about 160°C in this apparatus while it was pumped by a rotary pump protected by a trap for water which was cooled by liquid nitrogen. This drying process took about 6 hours, after which the product was crushed to yield a powder. The recovered dry solids weighed about 80 g and analysis showed that it contained 29.0% Si, 14.2% Cu and 0.06% Na.

This dry solid which was the catalyst precursor was reduced by heating to 450°C in a stream of gas composed of 10% by volume of hydrogen in nitrogen for five hours. After reduction the catalyst was ready for use in a hydrogenation reaction.

4.6 g of reduced catalyst was added from the quartz tube in which the reduction had been conducted to a stainless steel autoclave of approximately 1 litre capacity containing 250 ml of an aqueous solution comprising 125 g D-fructose in deionised water. The autoclave head was placed in position and the autoclave sealed and flushed with hydrogen gas before pressurizing with hydrogen to 103 bars. The vessel was equipped with a "hollow stirrer" stirring mechanism (a "Dispersimax" type agitator) which was run at approximately 1000 rpm. in order to ensure efficiency in mixing the contents of the autoclave. After pressurizing, the stirring mechanism was turned on and heating begun. The autoclave reached its reaction temperature of 100°C after approximately 20 minutes, at which time, designated as 0-hour, a sample was taken from the autoclave for analysis and pH measurement. The reaction mixture was sampled by bleeding off from the autoclave, while it was still under pressure, approximately 7 ml of the liquid mixture in two parts. The first was retained and the second analysed in the same way as described in Example 1. A pH measurement was made on this second part. During the time the sample was taken from the autoclave the stirrer was switched off.

After 2.5 hours at the reaction temperature of 100°C a further sample was taken for analysis and pH measurement and the autoclave and its contents were allowed to cool before depressurization and removal of the autoclave head. All the remaining reaction mixture and catalyst particles were removed and retained. The head and interior of the autoclave were washed thoroughly with deionised water and the washings combined with the recovered reaction mixture and all the samples which had been taken throughout the reaction.

These combined mixtures were filtered through a Pyrex sintered-funnel to remove the catalyst particles, which themselves were further washed with deionised water to wash through as much carbohydrate as possible.

The colourless filtrate was evaporated to dryness on a rotary evaporator and the remaining material was dried to constant weight in a vacuum oven at 80°C in order to give the recovered carbohydrate figures below. Details of the samples taken at the beginning and end of the period at the reaction temperature of 100°C are given in the Table. Mannitol and sorbitol were the only hexitol products.

| Time (hr) at 100°C | pH of sample | % D-Mannitol in hexitol products | % Conversion (approx) |
|---|---|---|---|
| 0 | 6.3 | 71.6 | 30 |
| 2.5 | 5.5 | 71.1 | 97 |

Recovered carbohydrate (constant weight)=122.3 g.

Example 14

The reaction was carried out as in Example 13 except that 125 g of L-sorbose was used in place of D-fructose, 4.7 g of reduced catalyst was used and the time at the reaction temperature of 100°C was 3 hours.

The following table gives the analyses and pH measurements of samples taken. Sorbitol and iditol were the hexitol products.

| Time (hr) at 100°C | pH of sample | % D-Mannitol in hexitol products | % Conversion (approx) |
|---|---|---|---|
| 0 | 6.7 | | 17 |
| 3 | 5.3 | 69.5 | 98 |

Recovered carbohydrate (constant weight)=125.0 g.

Example 15

A batch of catalyst was prepared essentially as in "Catalyst Preparation and Characterisation" except that 80 g cupric nitrate trihydrate, 60 g ammonium bicarbonate and 100 g Davison 952 silica were used. The final pH of the precipitation mixture was 7.0.

6 g of the dry solid from this preparation was reduced as described in "Catalyst Preparation and Characterisation" at 450°C.

4.4 g of reduced catalyst was added to a 1 litre stainless steel autoclave and the reaction was then carried out as described in Example 13 with 125 g of D-fructose. Samples of the reaction mixture were taken in the manner described in Example 13.

The following Table describes the progress of the reaction.

| Time (hr) at 100°C | % D-mannitol in hexitol products | % Conversion (approx) | pH of solution |
|---|---|---|---|
| 0 | — | 9 | 6.6 |
| 1 | 68.7 | 54 | — |
| 2 | 67.9 | 77 | — |
| 3 | 68.1 | 88 | — |
| 4 | 67.7 | 95 | 5.6 |

Example 16

A catalyst was prepared by dissolving 137 g of copper nitrate in 600 ml of water and adding 88 g silica followed by the dropwise addition over one hour of a solution containing 230 g ammonium bicarbonate in 1200 ml water whilst the temperature was kept at 40°C. The solution was then refluxed for one hour at 70°C under reduced pressure, suction filtered and the solid residue washed with deionised water and dried under vacuum at ca 160°C for about 4 hours to give a 1st stage catalyst. To 44 g of this dried 1st stage catalyst fresh copper nitrate (86 g in 600 ml solution) was added and fresh ammonium bicarbonate solution (115 g/litre) added dropwise over an hour and the whole refluxed for a further hour. The solid residue was filtered off, washed well with deionized water and dried for 4 hours under vacuum at 160°C to give a 2nd stage catalyst. The second stage catalyst precursor was reduced in pure hydrogen at a temperature of 325°C and used to perform hydrogenation of invert sugar (50/50 glucose/fructose) at temperatures between 120°C—160°C under a pressure of hydrogen of 119.8 bars.

136.5 g invert sugar (90 g sugar solids) were mixed with 7.7 g of reduced catalyst in a nitrogen purged dry box. The pH of the slurry was 6.4. The slurry was transferred to a one litre autoclave as described in Example 12 but operated at 1300 rpm, purged with nitrogen three times, with hydrogen three times and pressured to 103 bars. The autoclave was heated to 120°C and reacted 0.5 hour, heated to 140°C and reacted 0.5 hour, and heated to 160°C and reacted for 0.5 hour. The autoclave was cooled to room temperature, vented and the product removed. After filtration to remove catalyst the polyol product was ion-exchanged and concentrated to about 60% solids for analysis. This run is designated as Run 16A.

Two additional runs, 16B and 16C, were carried out in the same manner as Run 16A with temperatures, times and catalyst amounts as follows:

Run B — 120°C for 2.0 hours, then 130°C for 2.0 hours, 3.9 g of catalyst.

Run C — 120°C, 140°C, and 160°C for 0.5 hours each, 7.7 g of catalyst.

The analysis of the remaining sugar and the hexitol products was as follows:

| Product Analysis | 16A | 16B | 16C |
|---|---|---|---|
| Total sugar remaining | 0.6 | 6.9 | 0.6 |
| Sorbitol | 70.2 | 59.0 | 63.0 |
| Mannitol | 32.8 | 35.2 | 32.1 |

For a non-selective hydrogenation the product analysis should be (100% conversion) Sorbitol 75% Mannitol 25%.

Example 17

50 ml of 3.2 M sodium silicate solution was mixed thoroughly with 500 ml deionised water. 5 g Davison 952 silica was added and mixed. Then a solution of 101 g cupric nitrate trihydrate in 500 ml water and was added dropwise with stirring. Then the mixture was heated to 70°C and a solution of 80 g ammonium bicarbonate in 500 ml deionised water was added dropwise with stirring. The slurry was boiled and stirred for a further 45 minutes. The green solid was filtered off and washed thoroughly with deionised water. Analysis of solid gave Na 0.19%, Cu 32.5%, Si 16.6%.

Portions of the above solid were reduced in a stream of hydrogen gas at various temperatures and used in the hydrogenation of invert sugar as shown in the Table below.

Three hydrogenation runs, 17A, 17B and 17C, were carried out in the same manner as Run 16C (Example 16) except that the invert sugar solution was maintained at 120°C for 0.5 hour, then at 140°C for 0.5 hour, then 160°C for 1 hour. Catalyst reduction temperautres, final pH values, and product analyses for each run are given in the table below.

| Run | 17A | 17B | 7C |
|---|---|---|---|
| Catalyst Usage, g. | 5.65 | 5.70 | 5.55 |
| Final pH | 4.8 | 4.5 | 4.8 |
| Catalyst reduction temp °C | 325 | 225 | 200 |
| Product Analysis | | | |
| Sorbitol | 61.4 | 63.3 | 63.0 |
| Mannitol | 31.0 | 32.3 | 31.8 |
| Total Sugar | 0.6 | 0.5 | 0.3 |
| Reducing Sugar | 0.25 | 0.11 | 0.03 |

Example 18

A 5.9 g catalyst sample prepared as in Example 16, reduced at 175°C was used. The invert sugar hydrogenation procedure was the same as in Run 16C. Fresh catalyst was used in Run 18A. The same catalyst was re-used in Runs 18B, 18C and 18D with fresh sugar solutions (64% invert sugar in water). Results are as follows:

| Run | 18A | 18B | 18C | 18D |
|---|---|---|---|---|
| Yield wt% | 97.2 | 93.1 | 92.8 | 85.4 |
| Final pH | 4.5 | 4.1 | 4.5 | 3.2 |
| Product analysis | | | | |
| Sorbitol | 64.0 | 61.6 | 58.7 | 57.5 |
| Mannitol | 34.0 | 32.3 | 30.8 | 31.4 |
| Total sugar remaining | 0.7 | 3.4 | 3.1 | 6.7 |

Example 19

A catalyst was prepared in the same was as described in "Catalyst Preparation and Characterisation" except that 4.5 nickel nitrate and 30.8 g cupric nitrate trihydrate were used in place of 34.2 g cupric nitrate trihydrate. The analysis of the dried solid after precipitation, filtration and washing showed its contents to be 2.1% Ni, 15.6% Cu and 29.7% Si.

0.85 g of reduced catalyst, prepared from the dry solid by reduction as described in "Catalyst Preparation and Characterisation" at 450°C, was added to a Pyrex autoclave liner containing a solution of 10 g D-fructose in 25 ml of deionised water and a magnetic stirring bar coated with PTFE. The hydrogenation of the sugar was then carried out in the manner described in Example 1 except that the time of reaction at 100°C was 4 hours.

The analysis of the reaction mixture by the TMS method gave the % conversion as 97, and the hexitol products were analysed as

| % D-Mannitol | % D-glucitol (sorbitol) |
|---|---|
| 62 | 38 |

Example 20

A catalyst was prepared in the same way as described in "Catalyst Preparation and Characterisation" except that 17.1 g cupric nitrate trihydrate and 22.3 g nickel nitrate were used in place of 34.2 cupric nitrate trihydrate. The analysis of the dried solid after precipitation, filtration and washing showed its contents to be 10.9% Ni, 8.6% Cu and 29.1% Si.

0.2 g of reduced catalyst, prepared from the dry solid by reduction as described in "Catalyst Preparation and Characterisation" at 450°C was added to a Pyrex autoclave liner containing a solution of 10 g D-fructose in 25 ml of deionised water and a magnetic stirring bar coated with PTFE. The hydrogenation of the sugar was then carried out in the manner described in Example 1 except that the time of reaction at 100°C was 4 hours.

The analysis of the reaction mixture by the TMS method gave the % conversion as 93, and the hexitol products were analysed as

| % D-mannitol | % D-glucitol (sorbitol) |
|---|---|
| 59 | 41 |

Example 21

A dry greenish-blue solid was prepared as described in the section "Catalyst Preparation and Characterisation" and 20 g of this catalyst precursor before reduction was added with stirring to a solution of 1 g of chloroplatinic acid dissolved in 40 ml distilled water. The mixture was dried in an oven at 80°C and a dry solid obtained as a new catalyst precursor incorporating the platinum compound. This solid was then reduced at 450°C for 6 hours in a mixture of 10% hydrogen in nitrogen in order to produce a catalyst ready for hydrogenation reactions.

A solution containing 10 g fructose in 25 ml deionised water was treated with 0.5 g catalyst and a hydrogenation reaction carried out as described in Example 1 except that the time of reaction was 4 hours.

The conversion was measured as greater than 96% by the TMS method of analysis and by this same method the hexitol products were found to consist of 68% mannitol and 32% sorbitol.

**Claims**

1. A process for the production of a sugar alcohol by the hydrogenation of a keto-sugar which comprises contacting, in a pressurised system containing hydrogen gas at a temperature of at least 80°C, a solution of the keto-sugar with a supported catalyst comprising finely-divided metal and a particulate support material, characterised in that the keto-sugar is stereo-selectively hydrogenated by using a catalyst comprising finely-divided metallic copper and a particulate support material, and wherein the individual particles of copper have a maximum dimension of less than 60 nm.

2. A process as claimed in claim 1, wherein the individual particles of copper have a largest dimension of less than 35 nm.

3. A process as claimed in claim 1 or claim 2, wherein the hydrogenation is carried out in a solution of the keto-sugar in which the pH is not greater than 7.0.

4. A process as claimed in claim 3, wherein oxy-acids or oxy-anions of boron are present in the system during hydrogenation.

5. A process as claimed in any one of the preceding claims, wherein an oxygen-free inorganic anion is present in the system during hydrogenation.

6. A process as claimed in claim 5, wherein the inorganic anion is chloride, bromide or iodide.

7. A process as claimed in any one of the preceding claims, wherein the temperature of the hydrogenation is from 80°C to 200°C.

8. A process as claimed in any one of the preceding claims, wherein the copper is in the form of a mixture with a minor quantity of a Group VIII metal.

9. A process as claimed in any one of the preceding claims, wherein the support material is an insoluble solid substrate having a surface area from 5 to 1000 square metres per gram.

10. A process as claimed in claim 9, wherein the support material is silica or keiselguhr.

11. A process as claimed in any one of the preceding claims, wherein the catalyst is prepared by the reduction of a dispersed form of a compound of copper at an elevated temperature in the presence of the particulate support material.

12. A process as claimed in claim 11, wherein the reduction is conducted in an atmosphere containing hydrogen gas at a temperature in the range from 100°C to 500°C.

13. A process as claimed in any one of the preceding claims, wherein the keto-sugar comprises fructose, more than 50% of the fructose being converted in the process to mannitol.

14. A process as claimed in claim 1, wherein invert sugar, a mixture of equal parts of glucose and fructose is used as the reactant and the product of the process is a polyol mixture consisting essentially of mannitol and sorbitol, the weight of mannitol in the polyol mixture being greater than 50% of the fructose in the sugar mixture.

15. A process as claimed in any one of the preceding claims, wherein the process is repeated and the catalyst used for a first hydrogenation is re-used for subsequent reactions without regeneration.

**Revendications**

1. Procédé de production d'un sucre-alcool par hydrogénation d'un céto-sucre qui comprend la mise en contact, dans un système sous pression contenant de l'hydrogène gazeux à une température de plus de 80°C, d'une solution du céto-sucre avec un catalyseur sur support comprenant un métal finement divisé et une matière de support particulaire, caractérisé en ce que le céto-sucre est hydrogéné stéréo-sélectivement au moyen d'un catalyseur comprenant du cuivre métallique finement divisé et une matière de support particulaire et les particules individuelles de cuivre ont une dimension maximum de moins de 60 nm.

2. Procédé suivant la revendication 1, dans lequel les particules individuelles du cuivre ont une dimension maximum de moins de 35 nm.

3. Procédé suivant la revendication 1 ou 2, dans lequel l'hydrogénation est exécutée dans une solution du céto-sucre dans laquelle le pH n'est pas supérieur à 7,0.

4. Procédé suivant la revendication 3, dans lequel des oxacides ou des oxanions du bore sont présents dans le système pendant l'hydrogénation.

5. Procédé suivant l'une quelconque des revendications précédentes, dans lequel un anion inorganique exempt d'oxygène est présent dans le système pendant l'hydrogénation.

6. Procédé suivant la revendication 5, dans lequel l'anion inorganique est un anion chlorure, bromure ou iodure.

7. Procédé suivant l'une quelconque des revendications précédentes, dans lequel la température pour l'hydrogénation est de 80 à 200°C.

8. Procédé suivant l'une quelconque des revendications précédentes, dans lequel le cuivre se présente sous forme d'un mélange avec une quantité mineure d'un métal du groupe VIII.

9. Procédé suivant l'une quelconque des revendications précédentes, dans lequel la matière de support est un substrat solide insoluble ayant une surface spécifique de 5 à 1000 $m^2/g$.

10. Procédé suivant la revendication 9, dans lequel la matière de support est la silice ou le kieselguhr.

11. Procédé suivant l'une quelconque des revendications précédentes, dans lequel le catalyseur est préparé par réduction d'une forme dispersée d'un composé du cuivre à une température élevée en présence de la matière de support particulaire.

12. Procédé suivant la revendication 11, dans lequel la réduction est exécutée dans une atmosphère contenant de l'hydrogène gazeux à une température de l'intervalle de 100 à 500°C.

13. Procédé suivant l'une quelconque des revendications précédentes, dans lequel le céto-sucre comprend du fructose, le fructose étant converti pour plus de 50% en mannitol dans le procédé.

14. Procédé suivant la revendication 1, dans lequel du sucre inverti ou mélange de parties égales du glucose et de fructose est utilisé comme réactif et le produit du procédé est un mélange de polyols consistant essentiellement en mannitol et en sorbitol, le poids de mannitol dans le mélange de polyols étant supérieur à 50% du fructose dans le mélange de sucres.

15. Procédé suivant l'une quelconque des revendications précédentes, dans lequel le procédé est répété et le catalyseur utilisé pour une première hydrogénation est utilisé à nouveau pour des réactions ultérieures sans régénération.

**Patentansprüch**

1. Verfahren für die Herstellung eines Zuckeralkohols durch Hydrierung eines Ketozuckers, bei dem in einem druckdichten, gasförmigen Wasserstoff enthaltenden System bei einer Temperatur von mindestens 80°C eine Lösung des Ketozuckers mit einem Trägerkatalysator, der fein verteiltes Metall und ein teilchenförmiges Trägermaterial enthält, in Berührung gebracht wird, dadurch gekennzeichnet, daß der Ketozucker durch Einsatz eines Katalysators, der fein verteiltes, metallisches Kupfer und ein teilchenförmiges Trägermaterial enthält, wobei die einzelnen Teilchen des Kupfers eine größte Abmessung von weniger als 60 nm haben, stereoselektiv hydriert wird.

2. Verfahren nach Anspruch 1, bei dem die einzelnen Teilchen des Kupfers eine größte Abmessung von weniger als 35 nm haben.

3. Verfahren nach Anspruch 1 oder Anspruch 2, bei dem die Hydrierung in einer Lösung des Ketozuckers, deren pH-Wert nicht größer als 7,0 ist, durchgeführt wird.

4. Verfahren nach Anspruch 3, bei dem während der Hydrierung in dem System Oxosäuren oder Oxoanionen des Bors vorhanden sind.

5. Verfahren nach einem der vorhergehenden Ansprüche, bei dem während der Hydrierung in dem System ein sauerstofffreies, anorganisches Anion vorhanden ist.

6. Verfahren nach Anspruch 5, bei dem das anorganische Anion Chlorid, Bromid oder Jodid ist.

7. Verfahren nach einem der vorhergehenden Ansprüche, bei dem die Temperatur der Hydrierung 80°C bis 200°C beträgt.

8. Verfahren nach einem der vorhergehenden Ansprüche, bei dem das Kupfer in Form einer Mischung mit einer geringeren Menge eines Metalls der Gruppe VIII vorliegt.

9. Verfahren nach einem der vorhergehenden Ansprüche, bei dem das Trägermaterial ein unlöslicher, fester Träger mit einer spezifischen Oberfläche von 5 bis 1000 $m^2/g$ ist.

10. Verfahren nach Anspruch 9, bei dem das Trägermaterial Siliciumdioxid oder Kieselgur ist.

11. Verfahren nach einem der vorhergehenden Ansprüche, bei dem der Katalysator durch Reduktion einer fein verteilten Form einer Kupferverbindung bei einer erhöhten Temperatur in Gegenwart des teilchenförmigen Trägermaterials hergestellt worden ist.

12. Verfahren nach Anspruch 11, bei dem die Reduktion in einer gasförmigen Wasserstoff enthaltenden Atmosphäre bei einer Temperatur im Bereich von 100°C bis 500°C durchgeführt worden ist.

13. Verfahren nach einem der vorhergehenden Ansprüche, bei dem der Ketozucker Fructose enthält, wobei in dem Verfahren mehr also 50% der Fructose in Mannit umgewandelt werden.

14. Verfahren nach Anspruch 1, bei dem als Reaktionsteilnehmer Invertzucker, eine Mischung aus gleichen Teilen Glucose und Fructose, eingesetzt wird und bei dem das Produkt des Verfahrens eine im wesentlichen aus Mannit und Sorbit bestehende Mischung mehrwertiger Alkohole ist, wobei das Gewicht des Mannits in der Mischung mehrwertiger Alkohole größer als 50% des Gewichts der Fructose in der Zuckermischung ist.

15. Verfahren nach einem der vorhergehenden Ansprüche, wobei das Verfahren wiederholt wird und der für eine erste Hydrierung eingesetzte Katalysator ohne Regenerierung wieder für darauffolgende Reaktionen eingesetzt wird.